# EUROPÄISCHE PATENTSCHRIFT

(11) **EP 0 759 726 B1**
(45) Veröffentlichungstag und Bekanntmachung des Hinweises auf die Patenterteilung: **10.06.1998**
(21) Anmeldenummer: 96906728.9
(22) Anmeldetag: 01.03.1996
(51) Int. Cl.: A61B 17/70

(54) **ELEMENT ZUR HALSWIRBELSTABILISIERUNG**
CERVICAL VERTEBRA STABILISATION
ELEMENT DE STABILISATION DES VERTEBRES CERVICALES

(30) Priorität: 15.03.1995 DE 19509331
(43) Veröffentlichungstag der Anmeldung: 05.03.1997
(73) Patentinhaber: Harms, Jürgen, Prof. Dr., 76337 Waldbronn (DE); Biedermann, Lutz, 78048 Villingen-Schwenningen (DE)
(72) Erfinder: Harms, Jürgen, Prof. Dr., 76337 Waldbronn (DE); Biedermann, Lutz, 78048 Villingen-Schwenningen (DE)
(74) Vertreter: Prüfer, Lutz H., Dipl.-Phys.
(86) Internationale Anmeldenummer: EP9600883
(87) Internationale Veröffentlichungsnummer: WO9628105

(56) Entgegenhaltungen:
- WO-A-92/03100
- WO-A-94/26194

## Beschreibung

Die Erfindung betrifft ein Element zur Halswirbelstabilisierung.

Aus der WO-A-9426194 ist eine Knochenchirurgische Haltevorrichtung bekannt, die einen Grundkörper mit einem plattenförmigen Abschnitt mit einer sich senkrecht zur plattenebene erstreckenden Bohrung und einer Knochenschraube mit einem Gewindeabschnitt und einem Kopf, wobei sich der Gewindeabschnitt durch die Bohrung zu einer ersten Seite der Plattenebene erstreckt und der Kopf von dem plattenförmigen Abschnitt getragen wird, einem in der Plattenebene gegen die Bohrung seitlich versetzten zweiten Abschnitt mit einem Außengewinde und einem sich in Richtung der Gewindeachse erstreckenden Schlitz zur Aufnahme eines zu tragenden Stabes, und einer mit dem Außengewinde zusammenwirkenden Mutter zum Arretieren des einzulegenden Stabes, wobei ein Druckelement vorgehesen ist, aufweist.

Aus der DE-C-39 23 995 ist ein Stabilisierungselement zur Stabilisierung von Halswirbeln bekannt. Dieses umfaßt eine Platte, die durch Biegen an die gewünschte Form der Halswirbelsäule angepaßt wird. Anschließend werden eine Mehrzahl von Schrauben in die einander benachbarten Wirbel eingeschraubt. Stellt der Operateur dann fest, daß die Biegung nicht perfekt ist oder aus medizinischen Gründen noch geändert werden soll, müssen sämtliche Schrauben wieder herausgedreht werden, damit die Platte neu gebogen werden kann. Anschließend müßte ein erneutes Festschrauben erfolgen. Ein solches nochmaliges Einschrauben ist aus Stabilitätsgründen der Wirbel wegen der schwachen Verankerung im Knochen kaum möglich, so daß in der Praxis auf eine solche nachträgliche Korrekturausrichtung verzichtet werden muß.Aus der US 5,312,404 ist ein Element zur Wirbelstabilisierung bekannt, welches einen Grundkörper mit einem plattenförmigen Abschnitt und einer sich senkrecht zur Plattenebene erstreckenden Bohrung bekannt. Es ist eine Knochenschraube mit einem Gewindeabschnitt und einem Kopf vorgesehen, wobei sich der Gewindeabschnitt durch die Bohrung zu einer ersten Seite der Plattenebene erstreckt und die Knochenschraube mit dem Kopf von dem plattenförmigen Abschnitt getragen wird. Das Element weist neben dem plattenförmigen Abschnitt einen in der Plattenebene gegen die Bohrung seitlich versetzten zweiten Abschnitt auf. Der zweite Abschnitt besitzt eine Bohrung zur Aufnahme eines Stabes. Senkrecht zur Achse dieser Bohrung ist eine Gewindebohrung zur Aufnahme einer Schraube vorgesehen. Der Stab wird mittels einer darin einzuschraubenden Schraube gehalten.

Aus der US 5,360,429 ist ein Element zur Wirbelstabilisierung bekannt, welches aus einem Grundkörper mit einem plattenförmigen Abschnitt und einem seitlich versetzten zweiten Abschnitt steht. Der zweite Abschnitt enthält einen mit einem Außengewinde versehenen Schlitz, der zur Aufnahme eines zu tragenden Stabes dient. Es ist eine mit dem Außengewinde zusammenwirkende Mutter vorgesehen, die zum Arretieren des einzulegenden Stabes dient.

Aufgabe der Erfindung ist es, ein Element zur Halswirbelstabilisierung zu schaffen, bei dem der aufzunehmende Stab dauerhaft fixiert wird.

Diese Aufgabe wird durch das in Patentanspruch 1 gekennzeichnete Element zur Halswirbelstabilisierung gelöst. Weiterbildungen der Erfindung sind in den Unteransprüchen gekennzeichnet.

Weitere Merkmale und Zweckmäßigkeiten der Erfindung ergeben sich aus der Beschreibung eines Ausführungsbeispieles anhand der Figuren. Von den Figuren zeigen:
- Fig. 1: eine vergrößerte Schnittansicht entlang der Linie I-I in Fig. 2;
- Fig. 2: eine Draufsicht auf das in Fig. 1 gezeigte Element; und
- Fig. 3: eine Seitenansicht eines Teiles des in den Fig. 1 und 2 gezeigten Elementes.

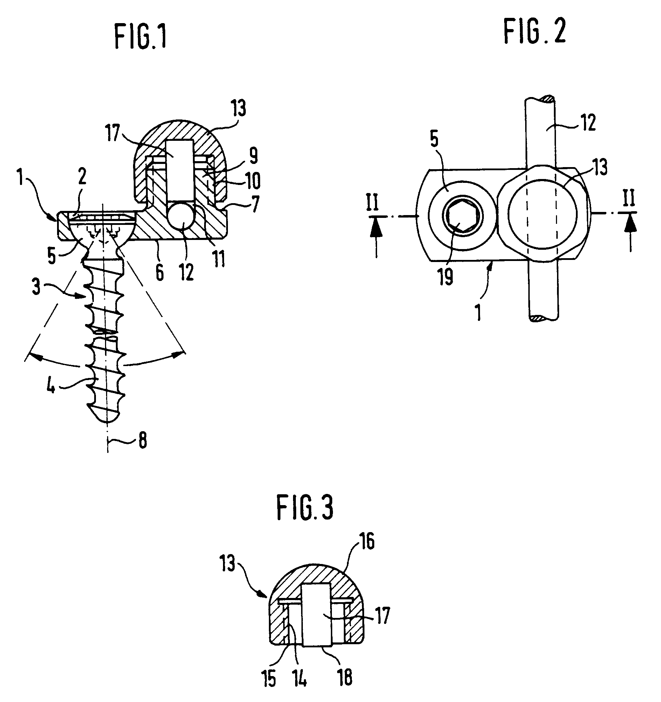

Wie an besten aus den Figuren 1 und 2 ersichtlich ist, weist das Element einen plattenförmigen Grundkörper 1 auf, der eine im wesentlichen rechteckige Form besitzt. Etwa im Zentrum seiner einen Hälfte weist der Grundkörper eine Bohrung 2 auf, die senkrecht zur Plattenebene ausgerichtet ist. In dieser Bohrung ist eine beispielsweise aus der genannten DE-C-39 23 995 bekannte Knochenschraube mit einem Gewindeabschnitt 4 und einem Kopf 5 angeordnet. Der Gewindeabschnitt 4 erstreckt sich durch die Bohrung in Richtung der ersten Seite 6 des plattenförmigen Grundkörpers 1. Auf der der ersten Seite 6 abgewandten zweiten Seite 7 des plattenförmigen Grundkörpers weist die Bohrung einen Rand in Form einer kugelsegmentförmigen Ausnehmung auf. Der Kopf 5 ist an seiner dem Gewindeabschnitt 4 zugewandten Unterseite entsprechend konvex kugelsegmentförmig ausgebildet. Die Radien von der kugelsegmentförmigen Ausnehmung und von dem entsprechenden konvex kugelsegmentförmigen Abschnitt des kugelsegmentförmigen Kopfes sind im wesentliche gleich und so aufeinander abgestimmt, daß die Knochenschraube innerhalb des durch die gestrichelten Linien angedeuteten kegelförmigen Bereiches um die Achse 8 herum zum bequemen Einschrauben in die Wirbel schwenkbar ist.

In seiner zweiten Hälfte weist der Grundkörper einen sich zu der zweiten Seite 7 hin erstreckenden zylindrischen Ansatz 9 auf. Der zylindrische Ansatz 9 weist ein Außengewinde 10 auf. Seine Symmetrie- bzw. Gewindeachse erstreckt sich senkrecht zur Plattenebene des Grundkörpers 1 bzw. parallel zur Achse der Bohrung 2.

Wie am besten aus Fig. 1 ersichtlich ist, erstreckt sich in einer durch die Symmetrieachse des zylindrischen Ansatzes 9 gehenden Symmetrieebene ein U-förmiger Schlitz 11 mit zwei zur Symmetrieachse parallenen Seitenwandungen und einem im wesentlichen zylinderabschnittförmigen Schlitzgrund. Der Schlitz dient zur Aufnahme eines Fixierstabes 12. Die Abmessung des Schlitzes und seines Grundes sind so gewählt, daß der Fixierstab 12 in den Schlitz und zu dessen Grund einführbar ist, ohne daß der Stab indessen ein seitliches Spiel besitzt.

Ferner ist eine Mutter 13 vorgesehen, die, wie am besten aus Fig. 3 ersichtlich ist, angrenzend an den Rand 15 einen Abschnitt mit einem Innengewinde 14 aufweist. Auf dem der Rand 15 gegenüberliegenden Rand weist die Mutter einen Hut 16 auf. Der Hut weist auf seiner dem das Innengewinde aufweisenden Abschnitt zugewandten Seite einen koaxial ausgerichteten Druckstift 17 auf. Der Druckstift 17 ist so bemessen, daß sein freies Ende 18 über den Rand 15 der Mutter 13 um Bruchteile eines Millimeters hervorsteht. Der Durchmesser des Stiftes ist gleich oder wenig kleiner als der Durchmesser des Stabes 12, so daß der Stift leicht in den Schlitz 11 ohne Behinderung einführbar ist, andererseits aber von diesem seitlich geführt wird.

Das Innengewinde 14 ist so ausgebildet, daß es mit dem Außengewinde 10 des zylindrischen Ansatzes 9 zusammenwirkt. Die Länge des Innengewindes 14 ist in Abhängigkeit von der axialen Länge des zylindrischen Ansatzes 9 und der Tiefe des Schlitzes 11 größer als die Schlitztiefe abzüglich des Durchmessers des einzulegenden Stabes 12.

Wie am besten aus Fig. 2 ersichtlich ist, weist die Knochenschraube 3 auf ihrer dem Gewindeabschnitt 4 abgewandten Seite des Kopfes eine Sechskantbohrung 19 zum Eindrehen der Schraube mittels eines Schraubendrehers auf.

Im Betrieb werden zunächst in die benachbarten Wirbel jeweils Grundkörper der oben beschriebenen Art mit Hilfe der Knochenschrauben 3 eingeschraubt, wobei die Knochenschrauben 3 noch nicht in ihre Endstellung festgeschraubt werden. Es wird dann der Fixierstab 12 in den jeweiligen Schlitz der einander benachbarten Grundkörper eingelegt. Anschließend wird die Hutmutter 13 aufgedreht. Dabei wird der Fixierstab 12 schon vor dem endgültigen Festdrehen der Mutter durch den wenig hervorstehenden Druckstift 17 in seiner Lage fixiert. Für den Fall einer gewünschten Änderung wird lediglich die Mutter wieder gelöst, der Stab herausgenommen und nachgebogen, wieder eingelegt und dann durch das Aufschrauben der Muttern über die Druckstifte wieder gehalten. Nach Erreichen der endgültigen Ausrichtung wird einerseits die Knochenschraube 3 fest angezogen. Andererseits wird durch das Anziehen der Mutter 13 eine endgültige Fixierung erreicht. Durch das Zusammenwirken des innen an den Wandungen des U-förmigen Schlitzes anliegenden Druckstiftes und der Mutter 13 erfolgt eine unlösbare feste Verbindung.

Mit der oben beschriebenen Konstruktion wird es möglich, auch in dem Bereich der Halswirbelsäule, bei der nur sehr wenig Platz für das Anbringen einer Fixationseinrichtung vorhanden ist, eine korrigierbare Ausrichtung und Fixation der so ausgerichteten Wirbel zu erreichen.

## Patentansprüche

1. Element zur Halswirbelstabilisierung, mit einem Grundkörper (1) mit einem plattenförmigen Abschnitt mit einer sich senkrecht zur Plattenebene erstreckenden Bohrung (2) und einer Knochenschraube (3) mit einem Gewindeabschnitt (4) und einem Kopf (5), wobei sich der Gewindeabschnitt (4) durch die Bohrung (2) zu einer ersten Seite (6) der Plattenebene erstreckt und der Kopf (5) von dem plattenförmigen Abschnitt getragen wird,
einem in der Plattenebene gegen die Bohrung (2) seitlich versetzten zweiten Abschnitt (9) mit einem Außengewinde (10) und einem sich in Richtung der Gewindeachse erstreckenden Schlitz (11) zur Aufnahme eines zu tragenden Stabes (12), und
einer mit dem Außengewinde (10) zusammenwirkenden Mutter (13) zum Arretieren des einzulegenden Stabes (12), wobei die Mutter (13) hutförmig ausgebildet ist und ein Druckelement (17) vorgesehen ist, wobei das Druckelement als Druckstift ausgebildet ist, der mit seinem einen Ende (18) bei aufgesetzter Mutter (13) in den Schlitz (11) hineinragt und mit seinem anderen Ende an der Mutter anliegt und dessen Durchmesser so gewählt ist daß der Druckstift (17) seitlich von den Seitenwandungen des Schlitzes gefürt ist.

2. Element zur Halswirbelstabilisierung nach Anspruch 1, dadurch gekennzeichnet, daß der Druckstift (17) über den Mutterrand (15) nach außen hervorsteht.

3. Element zur Halswirbelstabilisierung nach Anspruch 1 oder 2, dadurch gekennzeichnet, daß die Bohrung (2) auf der der ersten Seite (6) abgewandten zweiten Seite (7) der Plattenebene einen Rand in Form einer kugelsegmentförmigen Ausnehmung aufweist und daß der Kopf (5) an seiner dem Gewindeabschnitt (4) zugewandten Unterseite entsprechend konvex kugelsegmentförmig ausgebildet ist.

4. Element zur Halswirbelstabilisierung nach einem der Ansprüche 1 bis 3, dadurch gekennzeichnet, daß Mutter (13) und Druckstift (17) einstückig ausgebildet sind.

5. Element zur Halswirbelstabilisierung nach einem der Ansprüche 1 bis 4, dadurch gekennzeichnet, daß sich die Gewindeachse senkrecht zur Plattenebene erstreckt.

## Claims

1. Element for stabilizing cervical vertebrae, having a basic body (1) with a plate-shaped section with a bore (2) extending perpendicularly to the plane of the plate, and a bone screw (3) with a threaded section (4) and a head (5), the threaded section (4) extending through the bore (2) to a first side (6) of the plane of the plate and the head (5) being borne by the plate-shaped section,
a second section (9) laterally offset in relation to the bore (2) in the plane of the plate and having a male thread (10) and a slot (11), extending in the direction of the thread axis, for receiving a rob (12) to be borne, and
a nut (13), cooperating with the male thread (10), for securing the rod (12) to be inserted, the nut (13) being constructed in the shape of a cap and a pressure element (17) being provided, the pressure element being constructed as a pressure pin, which with its one end (18) projects into the slot (11) when the nut (13) is mounted and with its other end bears against the nut, and the diameter of which is chosen such that the pressure pin (17) is laterally guided by the side walls of the slot.

2. Element for stabilizing cervical vertebrae according to Claim 1, characterized in that the pressure pin (17) protrudes outwardly beyond the edge (15) of the nut.

3. Element for stabilizing cervical vertebrae according to Claim 1 or 2, characterized in that the bore (2), on the second side (7), facing away from the first side (6), of the plane of the plate, has an edge in the shape or a spherical-segment-shaped recess, and in that the head (5), at its underside facing the threaded section (4), is constructed correspondingly in the shape of a convex spherical segment.

4. Element for stabilizing cervical vertebrae according to one of Claims 1 to 3, characterized in that nut (13) and pressure pin (17) are constructed in one piece.

5. Element for stabilizing cervical vertebrae according to one of Claims 1 to 4, characterized in that the thread axis extends perpendicularly to the plane of the plate.

## Revendications

1. Elément destiné à la stabilisation des vertèbres cervicales, comportant un corps de base (1) qui présente une section en forme de plaque munie d'un trou (2) s'étendant perpendiculairement au plan de la plaque et une Vis d'ostéosynthèse (3) présentant une section filetée (4) et une tête (5), ladite section filetée (4) s'étendant à travers le trou (2) en direction d'un premier côté (6) du plan de la plaque et la tête (5) étant supportée par la section en forme de plaque,
une deuxième section (9) décalée latéralement dans le plan de la plaque vers le trou (2) et comportant un filetage extérieur (10) et une fente (11) s'étendant en direction de l'axe du filetage pour la réception d'une broche (12), et un écrou (13) qui coopère avec le filetage extérieur (10) afin de bloquer ladite broche (12), l'écrou (13) étant réalisé en forme de chapeau et un élément de compression (17) étant prévu, lequel est réalisé sous la forme d'une tige de compression, qui pénètre par l'une de ses extrémités (18), l'écrou (13) étant mis en place dans la fente (11) et s'appliquant par son autre extrémité sur ledit écrou et dont le diamètre a été sélectionné de telle manière que ladite tige de compression (17) est guidée latéralement par les parois latérales de la fente.

2. Elément destiné à la stabilisation des vertèbres cervicales selon la revendication 1, caractérisé en ce que la tige de compression (17) déborde vers l'extérieur au-delà du bord (15) de l'écrou.

3. Elément destiné à la stabilisation des vertèbres cervicales selon la revendication 1 ou 2, caractérisé en ce que le trou (2) présente sur le deuxième côté (7) ,opposé au premier côté (6) du plan de la plaque, un bord en forme d'un évidement de segment sphérique et que la tête (5) est réalisée en forme de segment sphérique de convexité correspondante sur sa face inférieure orientée vers la section filetée (4).

4. Elément destiné à la stabilisation des vertèbres cervicales selon l'une des revendications 1 à 3, caractérisé en ce que l'écrou 13 et la tige de compression (17) sont réalisés en une seule pièce.

5. Elément destiné à la stabilisation des vertèbres cervicales selon l'une des revendications 1 à 4, caractérisé en ce que l'axe du filetage s'étend perpendiculairement au plan de la plaque.
